# EUROPEAN PATENT SPECIFICATION

(11) **EP 3 701 922 B1**
(45) Date of publication and mention of the grant of the patent: **07.09.2022**
(21) Application number: 18895422.6
(22) Date of filing: 07.12.2018
(51) Int. Cl.: A61F 13/49, A61F 13/494, A61F 13/496, A61F 13/534

(54) **UNDERPANTS-SHAPED ABSORBENT ARTICLE**
SAUGFÄHIGE UNTERHOSE
ARTICLE ABSORBANT DE TYPE CULOTTE

(30) Priority: 28.12.2017 JP 2017254968
(43) Date of publication of application: 02.09.2020
(73) Proprietor: Unicharm Corporation, Ehime 799-0111 (JP)
(72) Inventor: NAGAI, Takahito, Kanonji-shi, Kagawa 769-1602 (JP); TANAKA, Suguru, Kanonji-shi, Kagawa 769-1602 (JP)
(74) Representative: Dolleymores
(86) International application number: PCT/JP2018/045157
(87) International publication number: WO 2019/131068

(56) References cited:
- WO-A1-01/39714
- WO-A1-01/39714
- WO-A1-2017/212886
- WO-A1-2017/212886
- JP-A- S63 235 501
- JP-A- 2017 217 159
- JP-A- 2018 153 389
- US-A1- 2005 038 404
- US-B2- 9 220 641

## Description

### [Technical Field]

The present invention relates to an underpants-shaped absorbent article.

### [Background Art]

A pull-on disposable diaper is known as one type of underpants-shaped absorbent article on absorbent article. Patent Document 1 discloses a pull-on disposable diaper that includes an outer sheet member and an absorbent main body that is joined to the skin surface side of the outer sheet member, and is also provided with rising portions (leak-proof wall portions) in which portions of a side sheet extending from the two widthwise side end portions of the absorbent main body rise upward due to contraction of raising elastic members.

The outer sheet member in Patent Document 1 is approximately hourglass-shaped in a plan view, and leg openings are formed by portions cut out inward in the width direction. Also, leg elastic members are provided along the edges of the leg openings such that the diaper fits around the wearer's legs. The leg elastic members are inclined elastic members that are inclined toward the crotch side and inward in the width direction. Accordingly, due to the inclined elastic members, the diaper (absorbent main body) can be pulled up more easily when being put on.

### [Citation List]

### [Patent Literature]

[Patent Document 1] Japanese Patent Application Publication No. 2010-94334

EP 3449883 A1 discloses an underpants-shaped absorbent article including an absorbent main body having a pair of leak-proof walls. The pair of leak-proof walls each have a skin-side portion that includes a plurality of elastic members and a non-skin-side portion that is arranged on a non-skin side of the skin-side portion.

The pair of leak-proof walls each have a joining portion in which at least portions of mutually opposing surfaces of the skin-side portion and the non-skin-side portion are joined.

EP 1153588 A1 discloses a disposable diaper in which a first absorbent body is provided between an outer sheet and a skin-side sheet. Side sheets are provided at opposite sides of an upper surface of the skin-side sheet and are adhered to the skin-side sheet. Raisable gathers and leg gathers are simultaneously formed at the respective side sheets by providing elastic threads in a specified relationship. A pocket is formed between the skin-side sheets and a second absorbent body is exchangeably inserted in the pocket.

### [Summary of Invention]

### [Technical Problem]

However, in order to provide the inclined elastic members in the portion of the outer sheet member that comes into contact with the front side of the wearer (hereinafter, the "front waist portion"), it is necessary to extend the length of the front waist portion toward the crotch side. For this reason, a pull-on disposable diaper has also been proposed, for example, which includes a front waist portion that is rectangular in a plan view and does not have inclined elastic members. With such a diaper, the lifting of the wearer's legs during walking is not inhibited by the front waist portion, thus allowing the wearer to walk easily. However, because inclined elastic members are not provided on the front side, it is not sufficiently easy to pull up the front side of the diaper.

The present invention was achieved in light of conventional problems such as that described above, and an aspect of the present invention is to improve the ability to pull up the front side of an underpants-shaped absorbent article that includes a front waist portion that is not provided with inclined elastic members.

### [Solution to Problem]

The present invention provides the underpants-shaped absorbent article of independent claim 1. The dependent claims specify preferred but optional features.

A main aspect of the present invention for achieving the above-described aspect is an underpants-shaped absorbent article having a vertical direction, a lateral direction, and a front-back direction that intersect each other,
the underpants-shaped absorbent article including:
   an absorbent main body that is folded in the front-back direction at a vertically lower end portion,
   the absorbent main body including an absorbent core, a skin-side sheet and a pair of leak-proof wall portions,
   the absorbent core being arranged extending in the vertical direction,
   the skin-side sheet being arranged on a wearer skin side with respect to the absorbent core;
a front waist portion that is joined to an upper end portion of the absorbent main body on a front side in the front-back direction; and
   a back waist portion that is joined to an upper end portion of the absorbent main body on a back side in the front-back direction,
   a lower end portion of the front waist portion not including an inclined elastic member that is inclined downward and inward in the lateral direction,
   the absorbent main body including an elastic member in two lateral side portions of the absorbent main body,
      the elastic member being capable of stretching and contracting in the vertical direction,
   each of the pair of leak-proof wall portions being capable of rising up on the skin side due to the elastic member,
   the absorbent main body including a front-end-portion fixed portion in the upper end portion on the front side,
      the front-end-portion fixed portion fixing each of the leak-proof wall portions in a manner that the leak-proof wall portion is not capable of rising up,
   the absorbent main body including a back-end-portion fixed portion in the upper end portion on the back side,
      the back-end-portion fixed portion fixing each of the leak-proof wall portions in a manner that the leak-proof wall portion is not capable of rising up,
   the absorbent main body including an intermittent fixed portion at a position spaced apart from the front-end-portion fixed portion and the back-end-portion fixed portion in the vertical direction,
      the intermittent fixed portion fixing each of the leak-proof wall portions to the skin-side sheet,
in a state where the underpants-shaped absorbent article is unfolded and stretched in the vertical direction, a vertical center of the intermittent fixed portion is on a front-waist-portion side with respect to a vertical center of the underpants-shaped absorbent article.

Features of the present invention other than the above will become clear by reading the description of the present specification with reference to the accompanying drawings.

### [Advantageous Effects of Invention]

According to the present invention, it is possible to improve the ability to pull up the front side of an underpants-shaped absorbent article that includes a front waist portion that is not provided with inclined elastic members.

### [Brief Description of the Drawings]

FIG. 1 is a front view of a pull-on disposable diaper 1.
FIG. 2 is a schematic plan view of the diaper 1 in an unfolded and stretched state.
FIG. 3 is a schematic cross-sectional view taken along a line I-I in FIG. 2.
FIG. 4 is a schematic cross-sectional view of the diaper 1 in an underpants-shaped state, taken along a front-back direction.
FIG. 5 is a diagram illustrating leak-proof wall portions 40.
FIG. 6 is a diagram illustrating the leak-proof wall portions 40.
FIG. 7A is a schematic cross-sectional view taken along a line I-I in FIG. 5, FIG. 7B is a schematic cross-sectional view taken along a line II-II in FIG. 5, and FIG. 7C is a schematic cross-sectional view taken along a line III-III in FIG. 5.
FIGS. 8A and 8B are schematic cross-sectional views of the leak-proof wall portions 40 in a put-on state.

### [Description of Embodiments]

At least the following matters will become clear with the description of this specification and the attached drawings. An underpants-shaped absorbent article having a vertical direction, a lateral direction, and a front-back direction that intersect each other,
the underpants-shaped absorbent article including:
an absorbent main body that is folded in the front-back direction at a vertically lower end portion,
   the absorbent main body including an absorbent core, a skin-side sheet and a pair of leak-proof wall portions,
   the absorbent core being arranged extending in the vertical direction,
   the skin-side sheet being arranged on a wearer skin side with respect to the absorbent core;
a front waist portion that is joined to an upper end portion of the absorbent main body on a front side in the front-back direction; and
a back waist portion that is joined to an upper end portion of the absorbent main body on a back side in the front-back direction,
a lower end portion of the front waist portion not including an inclined elastic member that is inclined downward and inward in the lateral direction,
the absorbent main body including an elastic member in two lateral side portions of the absorbent main body,
   the elastic member being capable of stretching and contracting in the vertical direction,
each of the pair of leak-proof wall portions being capable of rising up on the skin side due to the elastic member,
the absorbent main body including a front-end-portion fixed portion in the upper end portion on the front side,
   the front-end-portion fixed portion fixing each of the leak-proof wall portions in a manner that the leak-proof wall portion is not capable of rising up,
the absorbent main body including a back-end-portion fixed portion in the upper end portion on the back side,
   the back-end-portion fixed portion fixing each of the leak-proof wall portions in a manner that the leak-proof wall portion is not capable of rising up,
the absorbent main body including an intermittent fixed portion at a position spaced apart from the front-end-portion fixed portion and the back-end-portion fixed portion in the vertical direction,
   the intermittent fixed portion fixing each of the leak-proof wall portions to the skin-side sheet,
in a state where the underpants-shaped absorbent article is unfolded and stretched in the vertical direction, a vertical center of the intermittent fixed portion is on a front-waist-portion side with respect to a vertical center of the underpants-shaped absorbent article.

According to this underpants-shaped absorbent article, when the absorbent article is pulled up, the elastic member between the front-end-portion fixed portion and the intermittent fixed portion is likely to stretch forcefully. For this reason, the portion of the absorbent main body between the front-end-portion fixed portion and the intermittent fixed portion repeatedly stretch and contract, and the absorbent main body can be pulled up more easily. Accordingly, even if the front waist portion is not provided with the inclined elastic member, due to the intermittent fixed portion being provided shifted toward the front side, the portion of the absorbent main body on the front side can be pulled up more easily.

In such an underpants-shaped absorbent article,
the absorbent core has a narrow portion in a central portion in a longitudinal direction of the absorbent core,
a lateral width in the narrow portion is smaller than in end portions of the absorbent core,
at least a part of the intermittent fixed portion is arranged side-by-side with the narrow portion in the lateral direction and outward in the lateral direction with respect to the narrow portion.

According to this underpants-shaped absorbent article, the absorbent core can deform with a high degree of freedom, and the absorbent core can easily enter the crotch region of the wearer. The underpants-shaped absorbent article can therefore be pulled up smoothly.

In such an underpants-shaped absorbent article,
in the state where the underpants-shaped absorbent article is unfolded and stretched in the vertical direction,
the intermittent fixed portion is entirely on the front-waist-portion side with respect to the vertical center of the underpants-shaped absorbent article.

According to this underpants-shaped absorbent article, creases are less likely to be formed in the intermittent fixed portion. Accordingly, the portion of the absorbent main body where the intermittent fixed portion is located can be easily pulled up in a level manner while remaining a plate-shaped state, and the portion of the absorbent main body on the front side can be pulled up more easily.

In such an underpants-shaped absorbent article,
each of the leak-proof wall portions has a base region and a leading-end region,
   the base region being a region that extends from an inward end of the intermittent fixed portion toward a base of the leak-proof wall portion in the lateral direction,
   the leading-end region being a region that extends from the base region toward a leading end of the leak-proof wall portion, and
the leading-end region has a joining portion at a location laterally inward with respect to the leading end of the leak-proof wall portion,
   the joining portion being a portion in which at least a portion of mutually opposing surfaces are joined to each other.

According to this underpants-shaped absorbent article, the joining portion reduces the length of the sheet portions that can rise up at the side ends of the absorbent core. Accordingly, the portion of the absorbent core where the joining portion is located is less likely to sag, and can be pulled up more easily.

In such an underpants-shaped absorbent article,
in the state where the underpants-shaped absorbent article is unfolded and stretched in the vertical direction,
the joining portion is at a location not overlapped with the intermittent fixed portion in the vertical direction.

According to this underpants-shaped absorbent article, the intermittent fixed portion and the joining portion are arranged over a wider vertical range in the unfolded state, and the absorbent main body can be pulled up more easily.

In such an underpants-shaped absorbent article,
concerning a portion of the absorbent main body which is located between the front waist portion and the back waist portion and where at least either one of the intermittent fixed portion and the pinch joining portion are arranged,
in the state where the underpants-shaped absorbent article is unfolded and stretched in the vertical direction, a vertical length of the portion of the absorbent main body is longer than a vertical length of a remaining portion of the absorbent main body which is located between the front waist portion and the back waist portion.

Although it is difficult to pull up the portion of the absorbent main body between the front waist portion and the back waist portion, according to this underpants-shaped absorbent article, the intermittent fixed portion and the joining portion are arranged over a wider vertical range in the unfolded state, and the absorbent main body can be pulled up more easily.

In such an underpants-shaped absorbent article,
in the state where the underpants-shaped absorbent article is unfolded and stretched in the vertical direction,
a vertical length of the intermittent fixed portion is shorter than a vertical length of the joining portion.

According to this underpants-shaped absorbent article, the intermittent fixed portion is not unnecessarily long, thus making it possible to mitigate a degradation in the texture on the wearer's skin.

In such an underpants-shaped absorbent article,
the intermittent fixed portion is arranged inward with respect to lateral side ends of the absorbent main body.

According to this underpants-shaped absorbent article, the stiff intermittent fixed portion is not located at the lateral side end of the absorbent main body, thus allowing the lateral side end of the absorbent main body to be soft, and making it possible to improve the texture on the wearer's skin.

### Embodiment

Hereinafter, an underpants-shaped absorbent article according to the present invention will be described by way of example of a pull-on disposable diaper for adults. However, the underpants-shaped absorbent article according to the present invention can also be used as a pull-on disposable diaper for infants, sanitary shorts, or the like.

### Configuration of pull-on disposable diaper 1

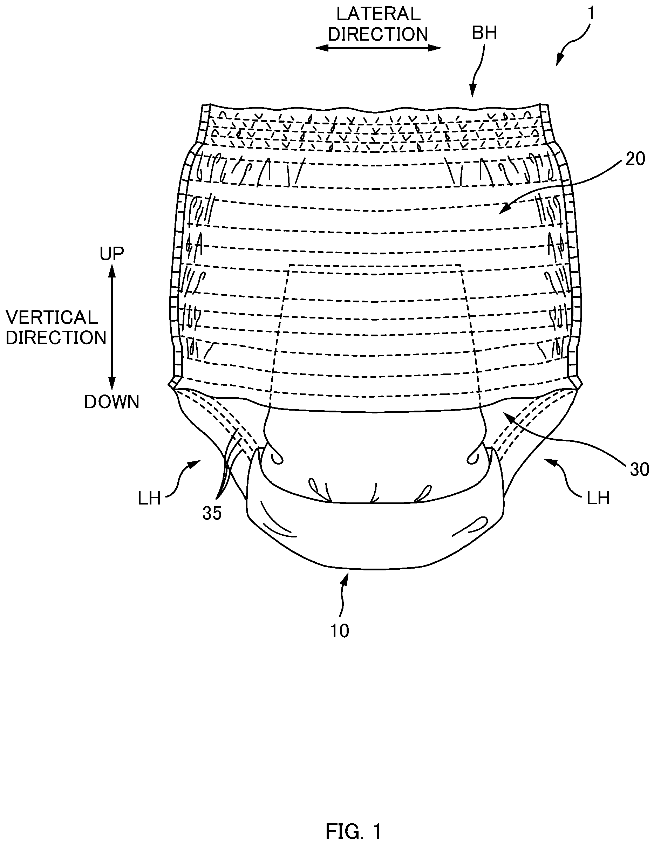

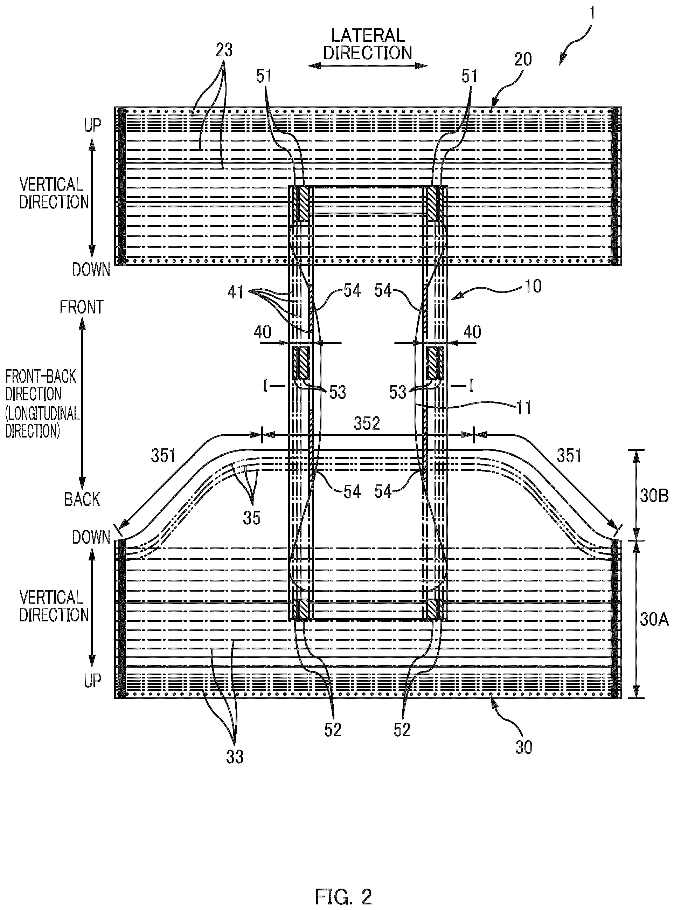

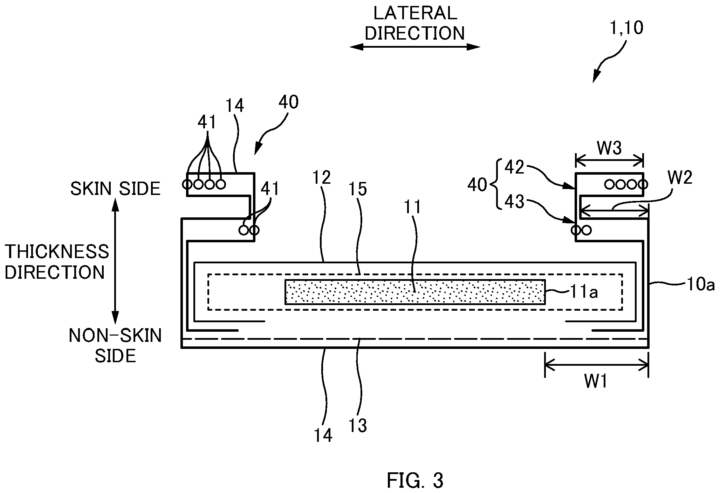

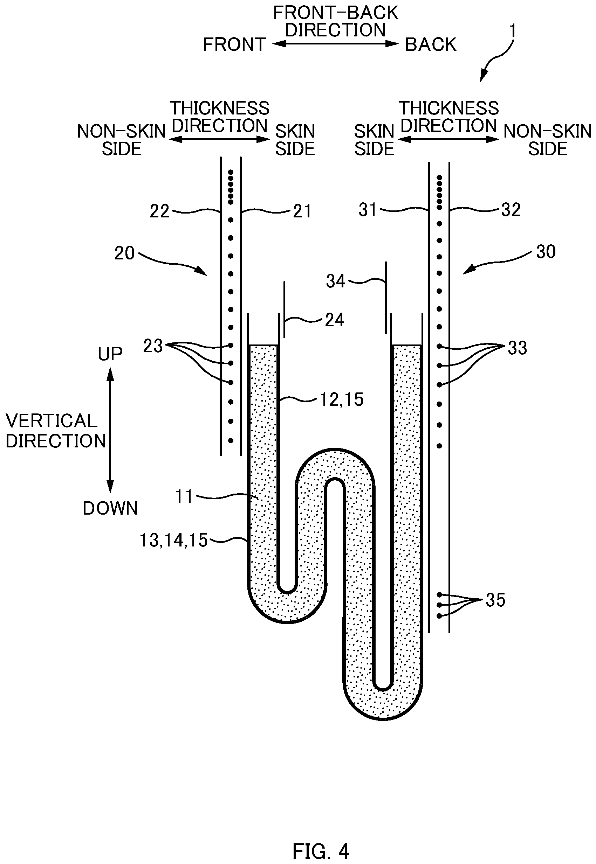

FIG. 1 is a front view of a pull-on disposable diaper 1 (hereinafter, "diaper"). FIG. 2 is a schematic plan view of the diaper 1 in an unfolded and stretched state. FIG. 3 is a schematic cross-sectional view taken along a line I-I in FIG. 2. FIG. 4 is a schematic cross-sectional view of the diaper 1 in an underpants-shaped state, taken along a front-back direction.

In the underpants-shaped state in FIG. 1, the diaper 1 has a vertical direction, a lateral direction, and a front-back direction that intersect each other, and also has a waist opening BH and a pair of leg openings LH. The upper side in the vertical direction corresponds to the waist opening BH side, and the lower side corresponds to the crotch side. Also, the front side in the front-back direction corresponds to the wearer's front side, and the back side corresponds to the wearer's back side. Also, as shown in FIG. 3, the direction in which constituent members of the diaper 1 are overlaid on each other will be called the thickness direction. With respect to the thickness direction, the side that comes into contact with the wearer is the skin side, and the opposite side is the non-skin side.

The diaper 1 includes an absorbent main body 10, a front waist portion 20, and a back waist portion 30. In the diaper 1 in the underpants-shaped state, the absorbent main body 10 (later-described absorbent core 11) is arranged extending along the vertical direction, and is folded in the front-back direction in a vertically lower end portion. Also, the front waist portion 20 is joined to the front upper end portion of the absorbent main body 10 from the non-skin side, and the back waist portion 30 is joined to the back upper end portion of the absorbent main body 10 from the non-skin side.

In the diaper 1 in the unfolded state in FIG. 2, the front waist portion 20 and the back waist portion 30 are arranged such that the longitudinal directions thereof conform to the lateral direction of the diaper 1. The longitudinal one-side end portion of the absorbent main body 10 is arranged at the lateral central portion of the front waist portion 20, and the longitudinal other-side end portion of the absorbent main body 10 is arranged at the lateral central portion of the back waist portion 30. The absorbent main body 10 in the unfolded state in FIG. 2 is then folded one time at approximately the longitudinal center, and the two lateral side portions of the front waist portion 20 are joined by welding or the like to the two lateral side portions of the back waist portion 30, thus obtaining the diaper 1 in the underpants-shaped state.

The absorbent main body 10 is provided with side elastic members 41 (elastic members of the present invention) that stretch and contract in the longitudinal direction (the vertical direction of the diaper 1), in the two lateral side portions. The absorbent main body 10 is also provided with a pair of leak-proof wall portions 40 that extend in the longitudinal direction, in the two lateral side portions. The leak-proof wall portions 40 can rise up on the skin side due to the side elastic members 41 (described in detail later).

The absorbent main body 10 also includes: an absorbent core 11 that absorbs excreted fluid; a liquid-permeable skin-side sheet 12 that is arranged on the wearer's skin side of the absorbent core 11; a liquid-impermeable non-skin-side sheet 13 that is arranged on the non-skin side of the absorbent core 11; and a sheet member 14. The two lateral side portions of the skin-side sheet 12 are folded back to the non-skin side so as to wrap around the absorbent core 11. The sheet member 14 is arranged on the non-skin side of the non-skin-side sheet 13, and forms the leak-proof wall portions 40 on the skin side of the skin-side sheet 12.

The absorbent core 11 is obtained by shaping liquid-absorbent fibers, such as pulp fibers containing a superabsorbent polymer (so-called SAP), into a predetermined shape. The absorbent core 11 of the present embodiment is approximately hourglass-shaped in a plan view, but is not limited to this shape, and may be rectangular in a plan view, for example. Also, the absorbent core 11 may be covered by a liquid-permeable core-wrapping sheet 15.

The front waist portion 20 and the back waist portion 30 respectively include inner-layer sheets 21 and 31, outer-layer sheets 22 and 32 arranged on the non-skin side of the inner-layer sheets 21 and 31, waist elastic members 23 and 33, and cover sheets 24 and 34. The waist elastic members 23 and 33 are arranged side-by-side in the vertical direction between the inner-layer sheets 21 and 31 and the outer-layer sheets 22 and 32, and are fixed in a laterally stretched state. The front waist portion 20 and the back waist portion 30 thus fit around the wearer's waist. Also, the cover sheets 24 and 34 cover the skin side of the upper end portion of the absorbent main body 10.

In the unfolded and stretched state in FIG. 2, the front waist portion 20 is rectangular in a plan view. The back waist portion 30 has an overlap portion 30A that is overlapped with the front waist portion 20 in the front-back direction, and an extension portion 30B that extends downward with respect to the front waist portion 20. The overlap portion 30A is rectangular in a plan view, and the extension portion 30B is shaped as an inverted trapezoid in a plan view. The wearer's buttocks can be covered by the extension portion 30B. On the other hand, the front waist portion 20 has a shorter vertical length than the back waist portion 30, and does not extend to the vicinity of the wearer's crotch. For this reason, leg movement is not hindered when the wearer lifts their legs toward their stomach while walking for example, and the wearer can walk easily.

Also, extension-portion elastic members 35 are provided extending along the lower end of the extension portion 30B of the back waist portion 30. Specifically, the extension-portion elastic members 35 each include a pair of inclined portions 351 that are inclined downward and laterally inward, and a straight portion 352 that extends in the lateral direction between the pair of inclined portions 351. Similarly to the waist elastic members 33, the extension-portion elastic members 35 are fixed in a stretched state between the inner-layer sheet 31 and the outer-layer sheet 32. Due to the extension-portion elastic members 35, the extension portion 30B fits to the wearer's buttocks, and curling of the extension portion 30B is suppressed.

### Leak-proof wall portion 40

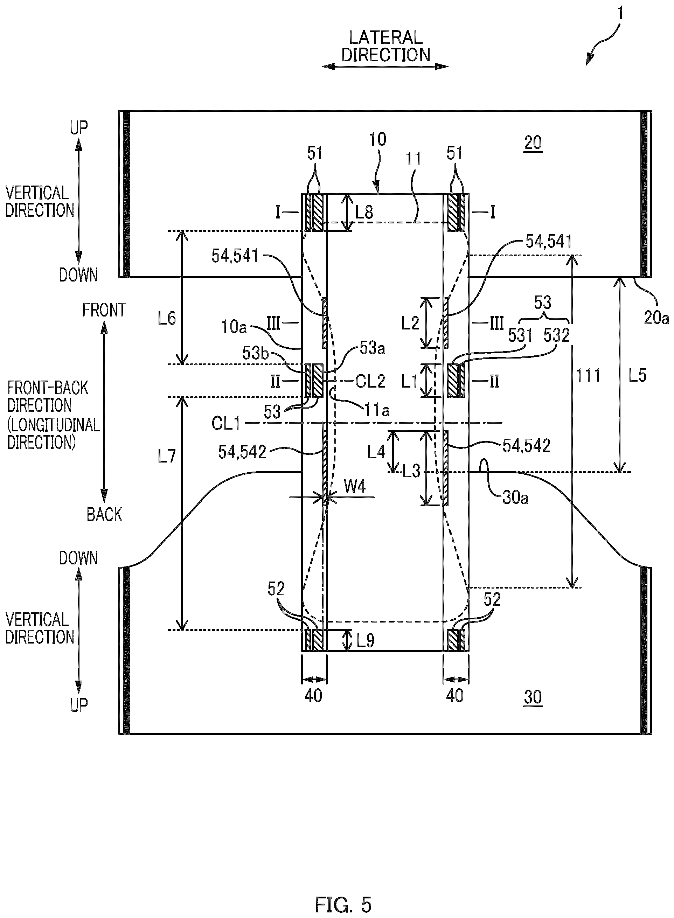

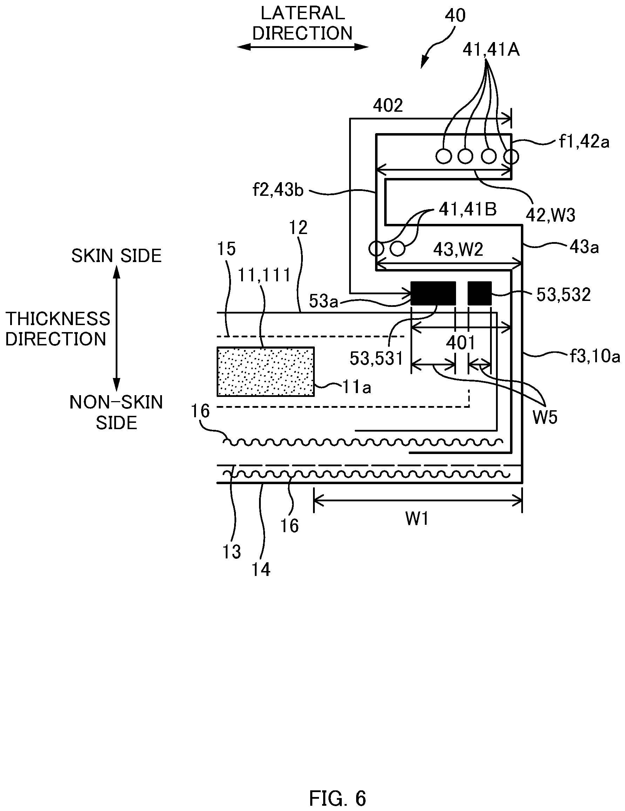

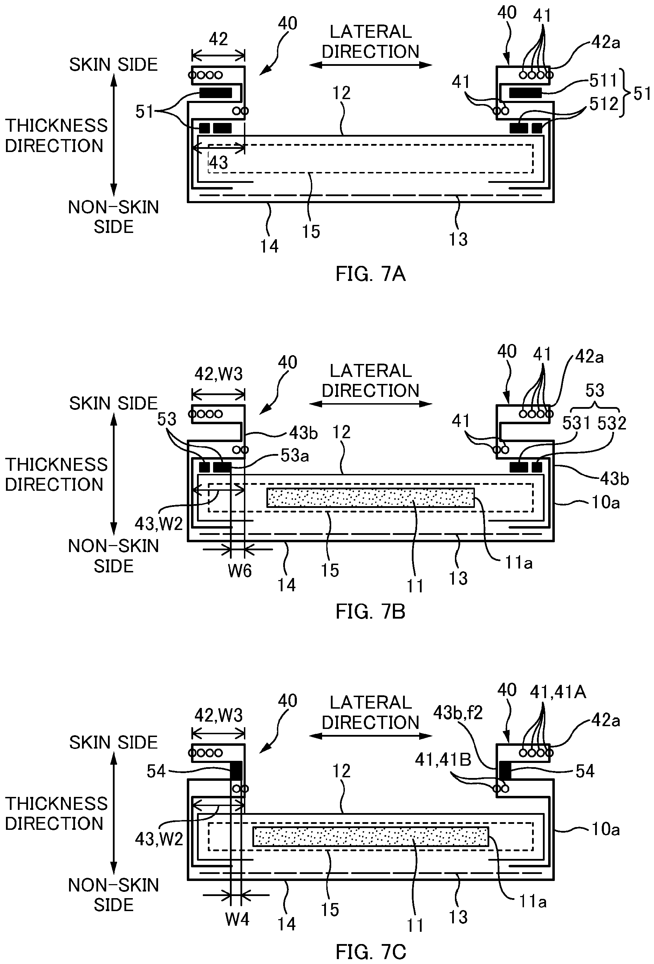

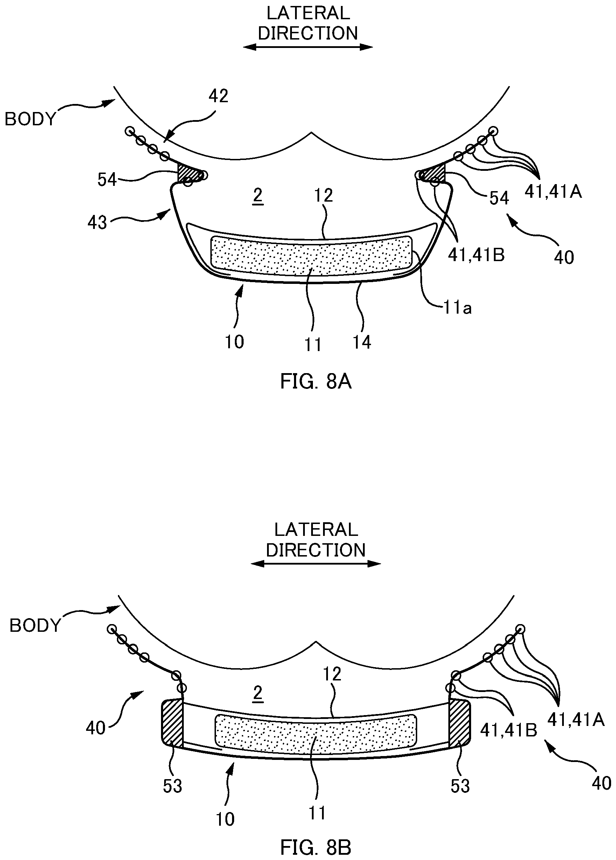

FIGS. 5 and 6 are diagrams illustrating the leak-proof wall portions 40. FIG. 5 is a schematic plan view of the diaper 1 in the unfolded and stretched state. FIG. 6 is a schematic cross-sectional view of the leak-proof wall portions 40. FIG. 7A is a schematic cross-sectional view taken along a line I-I in FIG. 5, FIG. 7B is a schematic cross-sectional view taken along a line II-II in FIG. 5, and FIG. 7C is a schematic cross-sectional view taken along a line III-III in FIG. 5. FIGS. 8A and 8B are schematic cross-sectional views of the leak-proof wall portions 40 in a put-on state.

In the following description, the state where the diaper 1 has been unfolded and stretched in the vertical direction refers to a state in which the front waist portion 20 and the back waist portion 30 have been detached, the diaper 1 has been unfolded in the longitudinal direction (vertical direction) of the absorbent main body 10, and the diaper 1 has been stretched in the longitudinal direction (vertical direction) of the absorbent main body 10 so as to eliminate wrinkles. Specifically, the diaper 1 has been stretched such that the lengths of the constituent members of the diaper 1 (the sheet member 14 and the like) match or are close to the dimensions of the members on their own.

### Configuration of leak-proof wall portion 40

The pair of leak-proof wall portions 40 provided in the absorbent main body 10 are each formed by folding the sheet member 14 that constitutes the exterior of the absorbent main body 10. The following describes an example of a method for forming the leak-proof wall portions 40.

First, the non-skin-side sheet 13 is placed on the sheet member 14, and then the two lateral side portions of the sheet member 14 are folded laterally inward on the skin side at folding lines f1 (see FIG. 6). The side elastic members 41 are then fixed between the two layers of the sheet member 14, in a state of being stretched in the longitudinal direction of the absorbent main body 10. Next, the two lateral side portions that include two layers of the sheet member 14 are folded laterally inward on the non-skin side at folding lines f2.

Lastly, the absorbent core 11 and the skin-side sheet 12 are placed on the non-skin-side sheet 13, and then the two lateral side portions that include four layers of the sheet member 14 are folded laterally inward on the skin side at folding lines f3. Accordingly, the portions of the sheet member 14 where the side elastic member 41 are fixed are arranged on the skin side with respect to the skin-side sheet 12. This obtains the leak-proof wall portions 40 that are folded so as to have an approximately S-shaped cross-section.

The leak-proof wall portions 40 each include: a "skin-side portion 42" that is located between the folding lines f1 and f2; and a "non-skin-side portion 43" that is located on the non-skin side with respect to the skin-side portion 42 and is between the folding lines f2 and f3. The skin-side portion 42 is folded laterally outward of the non-skin-side portion 43. Accordingly, a lateral outward end 42a of the skin-side portion 42 is a "leading end 42a of the leak-proof wall portion 40".

Also, the portion of the sheet member 14 that is located on the non-skin side with respect to the absorbent core 11 is fixed, with use of an adhesive 16 (see FIG. 6), to the back sheet 13 and the like up to the positions of lateral side ends 10a of the absorbent main body 10. Accordingly, the positions of the folding lines f3, which are the side ends 10a of the absorbent main body 10, are bases 43a of the leak-proof wall portions 40. In other words, a lateral outward end 43a of the non-skin-side portion 43 is a "base 43a of the leak-proof wall portion 40".

Four side elastic members 41A are provided side-by-side in the lateral direction between the two layers of the sheet member 14 that constitute each of the skin-side portions 42. The four side elastic members 41A are arranged shifted toward the leading end 42a of the leak-proof wall portion 40. Two side elastic members 41B are provided side-by-side in the lateral direction between the two layers of the sheet member 14 that constitute each of the non-skin-side portions 52. The two side elastic members 41B are arranged shifted toward a laterally inward end 43b of the non-skin-side portion 43.

Also, as shown in FIG. 5, in the absorbent main body 10, the upper end portion on the front side in the front-back direction of the diaper 1 includes "front-end-portion fixed portions 51" that fix the leak-proof wall portions 40 in a manner that the leak-proof wall portions 40 are not capable of rising up, and the upper end portion on the back side includes "back-end-portion fixed portions 52" that fix the leak-proof wall portions 40 in a manner that the leak-proof wall portions 40 are not capable of rising up.

Specifically, as shown in FIG. 7A, the front-end-portion fixed portions 51 include "skin-side fixed portions 511" in which the skin-side portion 42 and the non-skin-side portion 43 of the leak-proof wall portion 40 are fixed to each other with use of an adhesive or the like, and "non-skin-side fixed portions 512" in which the non-skin-side portion 43 of the leak-proof wall portion 40 and the skin-side sheet 12 are fixed to each other with use of an adhesive or the like. Although not illustrated, the back-end-portion fixed portions 52 also have a cross-section similar to that shown in FIG. 7A, and have skin-side fixed portions and non-skin-side fixed portions.

For this reason, the portions of the sheet member 14 that are between the front-end-portion fixed portions 51 and the back-end-portion fixed portions 52 in the longitudinal direction of the absorbent main body 10 are portions where the leak-proof wall portions 40 can rise. Also, when the wearer is in a sleeping posture for example, even if excreted fluid trapped by the leak-proof wall portions 40 flows to the front or back, the excreted fluid can be trapped by the front-end-portion fixed portions 51 and the back-end-portion fixed portions 52, thus making it possible to suppress leakage from the front and back.

Note that the width (length in the lateral direction) of the portions where the leak-proof wall portions 40 can rise is not a small amount in the present embodiment. Specifically, the width of the skin-side portions 42 and the width of the non-skin-side portions 43 are longer than 5 mm, not a small amount. Accordingly, if the two longitudinal end portions of the leak-proof wall portions 40 were not fixed in a manner that the leak-proof wall portion is not capable of rising up, the capable-of-rising-up portions of the leak-proof wall portions 40 that can rise up would contract toward the longitudinal center, making it impossible for the leak-proof wall portions 40 to rise up. In other words, providing the front-end-portion fixed portions 51 and the back-end-portion fixed portions 52 makes it possible to allow the leak-proof wall portions 40 to properly rise over a wide range in the longitudinal direction of the absorbent main body 10, and makes it possible for the leak-proof wall portions 40 to function.

Also, the absorbent main body 10 includes "intermittent fixed portions 53" in which the leak-proof wall portions 40 are fixed to the skin-side sheet 12, at positions that are spaced apart from the front-end-portion fixed portions 51 and the back-end-portion fixed portions 52 in the vertical direction. In other words, in a state where the diaper 1 is unfolded and stretched in the vertical direction (FIG. 5), on each side, the front-end-portion fixed portion 51, the intermittent fixed portion 53, and the back-end-portion fixed portion 52 are arranged intermittently in the vertical direction (the longitudinal direction of the absorbent main body 10).

Specifically, as shown in FIG. 6, in the intermittent fixed portions 53, the non-skin-side portions 43 of the leak-proof wall portions 40 are fixed to the skin-side sheet 12 with use of an adhesive or the like. The intermittent fixed portions 53 illustrated in FIG. 5 each include two fixed portions 531 and 532 that are rectangular in a plan view and are side-by-side in the lateral direction. However, there is no limitation to this, and the intermittent fixed portion 53 may be constituted by one fixed portion, or constituted by multiple fixed portions that are arranged side-by-side in the lateral direction or the vertical direction, for example.

The intermittent fixed portions 53 have a high stiffness due to the application of the adhesive, for example. For this reason, it is preferable that the intermittent fixed portions 53 are arranged inward with respect to the lateral side ends 10a of the absorbent main body 10. Due to not arranging the stiff intermittent fixed portions 53 at the lateral side ends 10a of the absorbent main body 10, it is possible to prevent the lateral side ends 10a (edges) of the absorbent main body 10 from irritating the wearer's thighs.

Also, as shown in FIG. 6, the leak-proof wall portions 40 each have a "base region 401", which is a region that extends from the position of an inward end 53a of the intermittent fixed portion 53 toward the base 43a of the leak-proof wall portion 40 (extends to the base 43a) in the lateral direction. Also, a "leading-end region 402" is a region that extends from the base region 401 toward the leading end 42a of the leak-proof wall portion 40 (extends to the leading end 42a).

As shown in FIG. 7C, the leading-end region 402 of each of the leak-proof wall portions 40 has a "pinch joining portion 54" (joining portion of the present invention) at a location laterally inward with respect to the leading end 42a of the leak-proof wall portion 40, and in the pinch joining portion 54, at least a portion of the mutually opposing surfaces are joined to each other. Specifically, in the pinch joining portion 54, portions of the skin-side portion 42 and the non-skin-side portion 43 are joined to each other with use of an adhesive or the like. Also, as shown in FIG. 5, the pinch joining portion 54 extends along the longitudinal direction of the absorbent main body 10.

The pinch joining portions 54 of the present embodiment are formed along the lateral inward ends 43b of the non-skin-side portion 43 (i.e., along the folding lines f2). However, there is no limitation to this, and the pinch joining portions 54 may be spaced apart from the lateral inward ends 43b of the non-skin-side portion 43 at locations laterally outward with respect to the lateral inward ends 43b.

The front-end-portion fixed portions 51, the back-end-portion fixed portions 52, the intermittent fixed portions 53, and the pinch joining portions 54 described above are formed by the application of an adhesive, but there is no limitation to this. For example, these portions may be formed using a pressure-adhesion means such as embossing.

In particular, in the intermittent fixed portion 53, there is a risk that a portion of the adhesive that forms the intermittent fixed portion 53 detach due to movement of the wearer's body. In the intermittent fixed portion 53, the portion of the leak-proof wall portion 40 that was folded laterally inward is fixed to the skin-side sheet 12. Accordingly, if a portion of the adhesive that forms the intermittent fixed portion 53 detaches, there is a risk that the adhesive becomes exposed to the wearer skin side, irritating the wearer's skin. For this reason, the intermittent fixed portion 53 may be formed with use of an adhesive, but is preferably formed by a pressure-adhesion means such as embossing.

### Improvement in ability to pull up diaper 1

As previously described, the back waist portion 30 (see FIG. 2) of the diaper 1 of the present embodiment includes the extension portion 30B that extends downward beyond the front waist portion 20, and the extension-portion elastic members 35 that extend along the lower end of the extension portion 30B. The inclined portions 351 of the extension-portion elastic members 35, which are inclined downward and laterally inward, extend for a certain length in the vertical direction. For this reason, when the diaper 1 is to be put on, as the user grips the waist portions 20 and 30 and pulls up the diaper 1, the stretching/contracting of the inclined portions 351 of the extension-portion elastic members 35 makes it easier to pull up the absorbent main body 10.

On the other hand, the lower end portion of the front waist portion 20 does not have the inclined elastic members that are inclined downward and inward in the lateral direction. In this case, the portion of the absorbent main body 10 on the front side is less easy to pull up than the portion of the absorbent main body 10 on the back side. Note that the inclined elastic members are not limited to being inclined with a straight shape as with the inclined portions 351 illustrated in FIG. 2, and may also include members that are inclined with various shapes such as an arc shape or a curved line shape.

In view of this, in the state where the diaper 1 is unfolded and stretched in the vertical direction (FIG. 5), it is desirable that the vertical center CL2 of the intermittent fixed portions 53 is arranged on the front waist portion 20 side relative to a product center CL1, which is the vertical center of the diaper 1. In other words, it is desirable that the intermittent fixed portions 53 are arranged shifted toward the front waist portion 20.

If the absorbent main body 10 were to not include the stiff intermittent fixed portions 53, the pull-up force applied by the user would be likely to act on the entirety of the absorbent main body 10, and the side elastic members 41 would overall stretch weakly.

In contrast, in the present embodiment, the pull-up force is likely to act on the vertically short portions between the stiff front-end-portion fixed portions 51 and the stiff intermittent fixed portions 53. For this reason, the portions of the side elastic members 41 between the front-end-portion fixed portions 51 and the intermittent fixed portions 53 stretch forcefully, and the contractive force also increases. Accordingly, the portions of the absorbent main body 10 between the front-end-portion fixed portions 51 and the intermittent fixed portions 53 repeatedly stretch and contract, and the absorbent main body 10 can be pulled up more easily. In other words, the portion of the absorbent main body 10 on the front side can be pulled up more easily.

Also, as shown in FIG. 7B, in the intermittent fixed portions 53, the skin-side sheet 12 is fixed to the leak-proof wall portions 40. For this reason, due to contraction of the side elastic members 41, lifted up are not only the leak-proof wall portions 40, but also the skin-side sheet 12 fixed to the leak-proof wall portions 40 and the absorbent core 11 fixed to the skin-side sheet 12 with use of an adhesive (not shown). This therefore improves the ability to easily pull up the portion of the absorbent core 11 on the front side where the intermittent fixed portions 53 are located.

More specifically, in the portion where the intermittent fixed portions 53 are not located, as shown in previously described FIG. 3, the sheet portion that can rise up at the lateral side end 11a of the absorbent core 11 has the following length. This length is the sum of a lateral length W1 of the sheet portion (the skin-side sheet 12, the non-skin-side sheet 13, and the sheet member 14) from the side end 11a of the absorbent core 11 to the side end 10a of the absorbent main body 10, a lateral length W2 of the non-skin-side portion 43 of the leak-proof wall portion 40, and a lateral length W3 of the skin-side portion 42 of the leak-proof wall portion 40 (W1 + W2 + W3). The sheet portion having this length (W1 + W2 + W3) is partially pinched by the intermittent fixed portions 53 as shown in FIG. 6. This therefore decreases the length of the sheet portion that can rise up at the side end 11a of the absorbent core 11. Accordingly, when the diaper 1 is pulled up, the portion of the absorbent core 11 where the intermittent fixed portions 53 are located is not likely to sag, and can be easily pulled up along with the leak-proof wall portions 40.

In this way, in the diaper 1 of the present embodiment, even if the inclined elastic members are not provided on the front side, the intermittent fixed portions 53 can make it easier to pull up the absorbent main body 10 on the front side. This therefore reduces a difference in the ability to easily pull up the absorbent main body 10 on the back side where the inclined elastic members (extension-portion elastic members 35) are provided and on the front side where the inclined elastic members are not provided. And, the diaper 1 can be pulled up smoothly.

Note that the back waist portion 30 is not required to have the inclined elastic members. For example, the back waist portion 30 may have the same rectangular shape in a plan view as the front waist portion 20. In this case as well, when the user puts on the diaper 1 on their own, they check how far the diaper 1 has been pulled up on the front side, and therefore improving the ability to easily pull up the diaper 1 on the front side makes it possible to give the user the impression that the diaper 1 is easy to pull up. Also, it is sufficient that the front waist portion 20 does not include the inclined elastic members, and there is no limitation to having a rectangular shape in a plan view. For example, the front waist portion 20 may have a planar shape similar to that of the back waist portion 30 shown in FIG. 2.

Also, as shown in FIG. 5, the absorbent core 11 of the present embodiment is approximately hourglass-shaped in a plan view, and has a narrow portion 111 in the longitudinal central portion, in which the lateral width is smaller than in the end portions. The narrow portion 111 can fit to the narrow crotch region of the wearer.

In the diaper 1 in the underpants-shaped state, it is preferable that at least a portion of the intermittent fixed portions 53 are arranged side-by-side with the narrow portion 111 of the absorbent core 11 in the lateral direction, and it is preferable that the intermittent fixed portions 53 are located on the lateral outward side with respect to the narrow portion 111. Specifically, it is preferable that when the diaper 1 is in the state of being unfolded and stretched in the vertical direction (FIG. 5), the narrow portion 111 of the absorbent core 11 and at least a portion of the intermittent fixed portions 53 are overlapped with each other in the vertical direction (the longitudinal direction of the absorbent main body 10), and that the intermittent fixed portions 53 are arranged outward in the lateral direction with respect to the side ends 11a of the absorbent core 11.

The intermittent fixed portions 53 may be located on the absorbent core 11, but in this case, the two lateral side portions of the absorbent core 11 are fixed to the pair of leak-proof wall portions 40 via the skin-side sheet 12. In this case, the absorbent core 11 has a low degree of freedom in terms of deformation, and the absorbent core 11 is pulled up in a level manner while remaining a plate-shaped state. Accordingly, in order for the narrow portion 111 of the absorbent core 11 to enter the narrow crotch region of the wearer, the user needs to pull the diaper 1 forcefully so as to cause the absorbent core 11 to bend into a mountain-like shape.

For this reason, it is preferable that the intermittent fixed portions 53 are arranged as previously described, fixing to the leak-proof wall portions 40 the sheet portions of the absorbent main body 10 where the absorbent core 11 is not located are fixed to the leak-proof wall portions 40 as shown in FIG. 7B. According to this configuration, the absorbent core 11 can deform with a high degree of freedom, and the narrow portion 111 of the absorbent core 11 can easily bend into a mountain-like shape to conform to the width of the crotch region of the wearer. Accordingly, the narrow portion 111 of the absorbent core 11 can easily enter the wearer's crotch region, and the diaper 1 can be pulled up smoothly.

Also, in the narrow portion 111 of the absorbent core 11, this elongates the length W1 (see FIG. 6) of the sheet portion from the side end 11a of the absorbent core 11 to the side end 10a of the absorbent main body 10, and elongates the length of the sheet portion that can rise up at the side end 11a of the absorbent core 11. For this reason, due to arranging the intermittent fixed portions 53 at positions corresponding to the narrow portion 111, the sheet portion that can rise up at the side end 11a of the absorbent core 11 is partially pinched, shortening the length of the sheet portion that can rise up. Accordingly, when the diaper 1 is pulled up, the narrow portion 111 of the absorbent core 11 is less likely to sag, and can easily be pulled up along with the leak-proof wall portions 40. In view of this as well, it can be said that the diaper 1 can be pulled up more easily.

Also, it is preferable that in the state where the diaper 1 is unfolded and stretched in the vertical direction (FIG. 5), the intermittent fixed portions 53 are entirely arranged on the front waist portion 20 side with respect to the product center CL1 of the diaper 1. In other words, it is preferable that the intermittent fixed portions 53 are not overlapped with the product center CL1 of the diaper 1.

When changing the diaper 1 from the unfolded state (FIG. 2) to the underpants-shaped state, the absorbent main body 10 is folded one time at the product center CL1. The stiff intermittent fixed portions 53 are likely to become creased if folded. In view of this, due to the intermittent fixed portions 53 being arranged as described above, the intermittent fixed portions 53 are not folded, and it is possible to prevent the intermittent fixed portions 53 from becoming creased. Accordingly, the portion of the absorbent main body 10 where the intermittent fixed portions 53 are located can be easily pulled up in a level manner while remaining a plate-shaped state due to the stiff intermittent fixed portions 53. This therefore improves the ability to easily pull up the portion of the absorbent main body 10 on the front side where the intermittent fixed portions 53 are located.

Also, as shown in FIG. 7C, the leak-proof wall portions 40 each have the pinch joining portions 54. Due to the pinch joining portions 54, the sheet portion that can rise up at the side end 11a of the absorbent core 11 is partially pinched, thus shortening the length of the sheet portion that can rise up. Accordingly, when the diaper 1 is pulled up, the portion of the absorbent core 11 where the pinch joining portions 54 are located is not likely to sag, and can be easily pulled up along with the leak-proof wall portions 40. The diaper 1 can therefore be pulled up more easily.

As described above, the diaper 1 (the absorbent main body 10) can be pulled up more easily due to the pinch joining portions 54 and the intermittent fixed portions 53. In view of this, it is preferable that in the state where the diaper 1 is unfolded and stretched in the vertical direction (FIG. 5), the pinch joining portions 54 are at locations not overlapped with the intermittent fixed portions 53 in the vertical direction. In other words, it is desirable that the pinch joining portions 54 and the intermittent fixed portions 53 are shifted from each other in the longitudinal direction of the absorbent main body 10. Accordingly, a wider range of the absorbent main body 10 in the longitudinal direction thereof can be pulled up more easily.

As shown in FIG. 5, the pinch joining portions 54 of the present embodiment include front pinch joining portions 541 that are arranged between the front-end-portion fixed portions 51 and the intermittent fixed portions 53, and back-pinch joining portions 542 that are arranged between the back-end-portion fixed portions 52 and the intermittent fixed portions 53. Due to the front pinch joining portions 541, the front-side portion of the absorbent main body 10 can be pulled up more easily. Also, due to the back-pinch joining portions 542, the back-side portion of the absorbent main body 10 can be pulled up more easily.

Also, in the state where the diaper 1 is unfolded and stretched in the vertical direction (FIG. 5), the vertical length of the intermittent fixed portions 53 is defined as a length L1, the vertical length of the front pinch joining portions 541 is defined as a length L2, and the vertical length of the back pinch joining portions 542 is defined as a length L3. Also, the vertical length of the portion of the back pinch joining portions 542 that is not overlapped with the back waist portion 30 is defined as a length L4. Moreover, defined as a length L5 is the vertical length of the portion of the absorbent main body 10 located between the front waist portion 20 and the back waist portion 30, that is to say the vertical length from the lower end 20a of the front waist portion 20 to the lower end 30a of the back waist portion 30.

The portion of the absorbent main body 10 between the front waist portion 20 and the back waist portion 30 is pulled up less easily than the portion overlapped with the front waist portion 20 or the back waist portion 30. In view of this, concerning a portion of the absorbent main body 10 which is located between the front waist portion 20 and the back waist portion 30 and where at least either the intermittent fixed portions 53 or the pinch joining portions 54 are arranged, it is preferable that in the state where the diaper 1 is unfolded and stretched in the vertical direction (FIG. 5), the vertical length of the portion of the absorbent main body 10 (L1 + L2 + L4) is longer than the vertical length of the remaining portion (L5 - (L1 + L2 + L4)) .

In other words, it is desirable that in the portion of the absorbent main body 10 between the front waist portion 20 and the back waist portion 30, the intermittent fixed portions 53 and the pinch joining portions 54 are arranged over a wide range in the longitudinal direction of the absorbent main body 10. Accordingly, the portion of the absorbent main body 10 that is not easily pulled up can be pulled up more easily.

Also, the contractive force (pull-up force) of the side elastic members 41 acts on the absorbent core 11 more easily in the portions where the intermittent fixed portions 53 are located than in the portions where the pinch joining portions 54 are located, and the absorbent core 11 can be pulled up more easily along with the leak-proof wall portions 40. In other words, compared with the pinch joining portions 54, the intermittent fixed portions 53 have a higher effect of allowing the absorbent main body 10 to be pulled up more easily.

Furthermore, in the present embodiment, a lateral length W5 of the intermittent fixed portions 53 (the sum of the lengths of the two fixed portions 531 and 532 that are arranged side-by-side in the lateral direction, as shown in FIG. 6) is longer than a lateral length W4 of the pinch joining portions 54 (see FIG. 6). For this reason, in the portions where the intermittent fixed portions 53 are located, the sheet portion that can rise up at the side end 11a of the absorbent core 11 has a shorter length, and the absorbent core 11 can be pulled up more easily along with the leak-proof wall portions 40. In other words, compared with the pinch joining portions 54, the intermittent fixed portions 53 have a higher effect of allowing the absorbent main body 10 to be pulled up more easily.

In view of this, it is desirable that in the state where the diaper 1 is unfolded and stretched in the vertical direction (FIG. 5), the vertical length L1 of the intermittent fixed portions 53 is shorter than the vertical lengths L2 and L3 of the pinch joining portions 54 (L1 < L2, L3) . In other words, it is preferable that the intermittent fixed portions 53 are not longer than necessary. According to this configuration, it is possible to mitigate a degradation in the texture on the wearer's skin caused by the stiff intermittent fixed portions 53. Also, even if the intermittent fixed portions 53 are not lengthened, the intermittent fixed portions 53 can further improve the ability to easily pull up the absorbent main body 10, thus making it possible to maintain the ability to easily pull up the diaper 1.

### Suppression of leakage of excrement

At the portions where the intermittent fixed portions 53 are located, the sheet portions that can rise up at the lateral side ends 11a of the absorbent core 11 have a short length. For this reason, even if the absorbent core 11 becomes heavy due to absorbing excreted fluid while the diaper 1 is put on, as shown in FIG. 8B, the portion of the absorbent core 11 where the intermittent fixed portions 53 are located is not likely to sag. In other words, the fit of the absorbent main body 10 to the wearer can be maintained even after excretion.

For this reason, even after excretion, the wearer does not feel awkwardness, and can walk easily for example. Also, the usage of the diaper 1 is less likely to be apparent. Moreover, even if excrement accumulates, the leakage of the excrement can be suppressed.

On the other hand, in the portions where the intermittent fixed portions 53 are located, the capable-of-rising-up portions of the leak-proof wall portions 40 that can rise up have a shorter length. For this reason, it is desirable that the intermittent fixed portions 53 are not provided over a wide range in the longitudinal direction of the absorbent main body 10, and that the intermittent fixed portions 53 are provided in only portions in the longitudinal direction of the absorbent main body 10. According to this configuration, the length of the capable-of-rising-up portions of the leak-proof wall portions 40 can be set longer between the front-end-portion fixed portions 51 and the intermittent fixed portions 53, and between the intermittent fixed portions 53 and the back-end-portion fixed portions 52.

More specifically, the length of the capable-of-rising-up portion of the leak-proof wall portion 40 in the intermittent fixed portion 53 (see FIG. 7B) is the sum of the lateral length W6 from the inward end 53a of the intermittent fixed portion 53 to the inward end 43b of the non-skin-side portion 43, and the lateral length W3 of the skin-side portion 42 (W6 + W3) .

In contrast, in the portions where the intermittent fixed portion 53 and the pinch joining portion 54 are not located, the length of the capable-of-rising-up portion of the leak-proof wall portion 40 (see FIG. 3) is the sum of the lateral lengths of the non-skin-side portion 43 and the skin-side portion 42 (W2 + W3). Also, in the portions where only the pinch joining portion 54 is located, the length of the capable-of-rising-up portion of the leak-proof wall portion 40 (see FIG. 7C) is obtained by subtracting the lateral length of the pinch joining portion 54 from the sum of the lateral lengths of the non-skin-side portion 43 and the skin-side portion 42 (W2 + W3 - W4).

Accordingly, in the portion where the intermittent fixed portions 53 are not located (e.g., FIG. 8A), it is possible to ensure a space 2 between the wearer's body and the absorbent main body 10 that is appropriately larger than in the portion where the intermittent fixed portions 53 are located (e.g., FIG. 8B). Excrement can be contained in the space 2, and it is possible to suppress the leakage of excrement. Also, male genitalia can be contained in the space 2 on the front side with respect to the intermittent fixed portions 53, and the wearer's buttocks and excrement can be contained in the space 2 on the back side with respect to the intermittent fixed portions 53.

Also, the absorbent main body 10 of the present embodiment includes the side elastic members 41 that stretch and contract in the vertical direction in the two lateral side portions, and the side elastic members 41 are arranged in only the leading-end regions 402 of the leak-proof wall portions 40. If the side elastic members 41 were arranged in the portions of the sheet member 14 that are fixed by the intermittent fixed portions 53, the contractive force of the side elastic members 41 would be partially suppressed by the stiffness of the intermittent fixed portions 53.

In view of this, as shown in FIG. 6, it is preferable that the side elastic members 41 are entirely located on leading-end-42a-of-leak-proof-wall-portion-40 side with respect to the lateral inward end 53a of the intermittent fixed portions 53. Accordingly, it is possible to prevent the case where the contractive force of the side elastic members 41 is suppressed by the intermittent fixed portions 53. Accordingly, the contractive force of the side elastic members 41 can be used effectively to cause the leak-proof wall portions 40 to reliably rise up so as to be in close contact with the wearer, and it is possible to suppress the leakage of excrement.

Note that there is no limitation to the configuration described above, and the side elastic members 41 may be arranged in the base region 401. Specifically, the side elastic members 41 may be arranged in a portion of the base region 401 that is not overlapped with the intermittent fixed portions 53 in the lateral direction, or is overlapped with the intermittent fixed portions 53.

Also, the absorbent core 11 of the present embodiment includes the narrow portion 111. The narrow portion 111 improves the fit of the absorbent core 11 to the wearer's crotch region, but the absorption capacity decreases due to the reduced width. For this reason, it is preferable that, as described above, in the diaper 1 in the underpants-shaped state, the intermittent fixed portions 53 are arranged so as to at least partially be side-by-side with the narrow portion 111 of the absorbent core 11 in the lateral direction and outward in the lateral direction with respect to the narrow portion 111.

According to this configuration, it is possible to prevent the case where the skin-side surface of the narrow portion 111 is covered by the leak-proof wall portions 40 and the adhesive of the intermittent fixed portions 53. In other words, it is possible to prevent a reduction in the absorbing surface area of the narrow portion 111, and to maintain the absorbing capability of the narrow portion 111. Accordingly, the leakage of excreted fluid can be suppressed.

Also, the wearer's urination position is located on the front side (front side) with respect to their crotch region. For this reason, as previously described, it is desirable that the intermittent fixed portions 53 are arranged shifted toward the front waist portion 20. According to this configuration, the intermittent fixed portions 53 are provided at locations that correspond to the portion of the absorbent core 11 that comes into contact with the excretion source. For this reason, the intermittent fixed portions 53 can suppress the sagging of the portion of the absorbent core 11 that becomes heaviest due to absorbing excreted fluid.

Also, due to the intermittent fixed portions 53 being arranged shifted toward the front waist portion 20, the following length relationship is satisfied. Specifically, in the state where the diaper 1 is unfolded and stretched in the vertical direction (FIG. 5), a vertical length L7 of the leak-proof wall portions 40 between the intermittent fixed portions 53 and the back-end-portion fixed portions 52 can be set longer than a vertical length L6 of the leak-proof wall portions 40 between the front-end-portion fixed portions 51 and the intermittent fixed portions 53 (L6 < L7).

According to this configuration, the back side can be provided with a long region in which the intermittent fixed portions 53 are not located, and in which the leak-proof wall portions 40 can rise up over a long range. For this reason, it is possible to ensure a sufficiently large space 2 (see FIG. 8A) for containing the wearer's buttocks and excrement. Also, excreted fluid is likely to flow to the back side when the wearer is in a sleeping posture for example, and excreted fluid that has flowed to the back side is blocked by the reliably risen-up leak-proof wall portions 40. Leakage on the back side can thus be reliably suppressed.

Furthermore, in the present embodiment, in the state where the diaper 1 is unfolded and stretched in the vertical direction (FIG. 5), a vertical length L9 of the back-end-portion fixed portions 52 is shorter than a vertical length L8 of the front-end-portion fixed portions 51 (L8 > L9).

For this reason, the vertical length L7 of the leak-proof wall portions 40 between the intermittent fixed portions 53 and the back-end-portion fixed portions 52 can be set longer than the vertical length L6 of the leak-proof wall portions 40 between the front-end-portion fixed portions 51 and the intermittent fixed portions 53. Accordingly, it is possible to ensure a sufficiently large space 2 for containing the wearer's buttocks and excrement, and to also suppress back leakage.

Also, as shown in FIG. 7C, the leak-proof wall portions 40 each have the pinch joining portions 54. The pinch joining portions 54 maintain the folded shape of the leak-proof wall portion 40, that is to say maintain the fold at the folding line f2. For this reason, in the state where the diaper 1 is put on (FIG. 8A), the non-skin-side portions 43 rise toward the wearer due to the side elastic members 41B arranged in the non-skin-side portions 43 of the leak-proof wall portions 40, and the skin-side portions 42 are in flat contact with the wearer due to the side elastic members 41A arranged in the skin-side portions 42.

Also, the pinch joining portions 54 are stiff, and the pinch joining portions 54 in particular maintain a flat shape in the skin-side portions 42, thus allowing the skin-side portions 42 to reliably come into close contact with the wearer. On the other hand, the portions of the skin-side portions 42 other than the pinch joining portions 54 have a low stiffness, and can come into close contact with the wearer as curved surfaces that conform to the protrusion/recession of the wearer's body. For this reason, it is possible to increase the extent of contact of the leak-proof wall portions 40 with the wearer, and to suppress the leakage of excrement.

Note that in the pinch joining portions 54, portions of the sheet member 14 that forms the leak-proof wall portions 40 are pinched, thus reducing the length of the capable-of-rising-up portions of the leak-proof wall portions 40. For this reason, it is preferable that in the state where the diaper 1 is unfolded and stretched in the vertical direction (FIG. 5), the pinch joining portions 54 are at locations not overlapped with the intermittent fixed portions 53 in the vertical direction.

According to this configuration, it is possible to prevent the case where the length of the capable-of-rising-up portions of the leak-proof wall portions 40 is excessively reduced due to the provision of both the pinch joining portions 54 and the intermittent fixed portions 53. Accordingly, the leak-proof wall portions 40 can appropriately rise up over a wide range in the longitudinal direction of the absorbent main body 10, and the leakage of excrement can be suppressed.

As shown in FIG. 5, in the present embodiment, the front pinch joining portions 541 are arranged between the front-end-portion fixed portions 51 and the intermittent fixed portions 53, and the back pinch joining portions 542 are arranged between the back-end-portion fixed portion 52 and the intermittent fixed portions 53.

For this reason, the space 2 (see FIG. 8A) for containing excrement on the front side with respect to the intermittent fixed portions 53 can be sealed by the front pinch joining portions 541, and the leakage of excrement can be suppressed. Similarly, the space 2 for containing excrement on the back side with respect to the intermittent fixed portions 53 can be sealed by the back pinch joining portions 542, and the leakage of excrement can be suppressed.

Furthermore, it is preferable that in the state where the diaper 1 is unfolded and stretched in the vertical direction (FIG. 5), the vertical length L3 of the back pinch joining portions 542 is longer than the vertical length L2 of the front pinch joining portions 541 (L3 > L2).

According to this configuration, the leak-proof wall portions 40 on the back side can reliably come into contact with the wearer due to the long back pinch joining portions 542. Also, excreted fluid is likely to flow to the back side when the wearer is in a sleeping posture for example, and excreted fluid that has flowed to the back side is reliably blocked by the reliably risen-up leak-proof wall portions 40. Leakage on the back side can thus be reliably suppressed.

Also, it is preferable that the lateral length W4 of the pinch joining portions 54 (see FIG. 5) is shorter than the lateral length W5 (W4 < W5) of the intermittent fixed portions 53 (the sum of the lengths of the two fixed portions 531 and 532 that are arranged side-by-side in the lateral direction, as shown in FIG. 6) . According to this configuration, the length of the capable-of-rising-up portions of the leak-proof wall portions 40 in the pinch joining portions 54 can be set longer than the length of the capable-of-rising-up portions of the leak-proof wall portions 40 in the intermittent fixed portions 53. Accordingly, in the portion where the pinch joining portions 54 are provided, it is possible to ensure a sufficiently large space 2 (see FIG. 8A) for containing excrement, and the leakage of excrement can be suppressed.

Also, the pinch joining portions 54 are provided in the leading-end regions 402 of the leak-proof wall portions 40 where the side elastic members 41 are arranged. For this reason, by reducing the lateral length W4 of the pinch joining portions 54, it is possible to reduce the number of side elastic members 41 that are arranged in the pinch joining portions 54.

Also, it is preferable that the number of side elastic members 41B arranged in the pinch joining portions 54 (two in the present embodiment) is smaller than the number of side elastic members 41 that are not arranged in the pinch joining portions 54 (four in the present embodiment) . In other words, it is preferable that the number of side elastic members 41 whose contractive force is partially suppressed by the stiffness of the pinch joining portions 54 is smaller than the number of side elastic members 41 whose contractive force is not suppressed by the pinch joining portions 54. According to this configuration, the contractive force of the side elastic members 41 can be used effectively to cause the leak-proof wall portions 40 to reliably rise up so as to be in close contact with the wearer. Accordingly, the leakage of excrement can be suppressed.

Also, it is preferable that in the state where the diaper 1 is unfolded and stretched in the vertical direction (FIG. 5), the pinch joining portions 54 are at locations not overlapped with the product center CL1 of the diaper 1.

According to this configuration, it is possible to prevent the case where the stiff pinch joining portions 54 are folded and become creased when changing the diaper 1 from the unfolded state (FIG. 2) to the underpants-shaped state. For this reason, the pinch joining portions 54 can come into close surface-to-surface contact with the wearer along the longitudinal direction of the absorbent main body 10, and the leakage of excrement can be suppressed.

### Other embodiments

Although the embodiment of the present disclosure has been described hereinabove, the above embodiment of the present disclosure is simply to facilitate understanding of the present disclosure and are not in any way to be construed as limiting the present disclosure. The present disclosure may variously be changed or altered without departing from its gist and encompass equivalents thereof.

Although the example of a diaper in which the front waist portion 20 and the back waist portion 30 are separate and independent members is described in the above embodiment, a configuration is possible in which a crotch portion is provided between the front waist portion 20 and the back waist portion 30, and the front waist portion 20 and the back waist portion 30 are a single continuous member.

Also, an aspect is possible in which the leak-proof wall portions 40 do not have the pinch joining portions 54. Also, an aspect is possible in which only the front pinch joining portions 541 or the back pinch joining portions 542 are provided. Furthermore, an aspect is possible in which the pinch joining portions 54 extend continuously in the longitudinal direction of the absorbent main body 10. Moreover, the relationship between the lengths of the intermittent fixed portions 53 and the pinch joining portions 54 or the like is not limited to the relationship described in the above embodiment.

### [Reference Signs List]

1 diaper (underpants-shaped absorbent article),
10 absorbent main body, 11 absorbent core, 111 narrow portion,
12 skin-side sheet, 13 non-skin-side sheet,
14 sheet member, 15 core-wrapping sheet,
20 front waist portion, 21 inner-layer sheet, 22 outer-layer sheet,
23 waist elastic member, 24 cover sheet,
30 back waist portion, 31 inner-layer sheet, 32 outer-layer sheet,
33 waist elastic member, 34 cover sheet, 35 extension-portion elastic member,
40 leak-proof wall portion, 401 base region, 402 leading-end region,
41 side elastic member (elastic member), 42 skin-side portion, 43 non-skin-side portion,
42a leading end of leak-proof wall portion, 43a base of leak-proof wall portion,
51 front-end-portion fixed portion, 52 back-end-portion fixed portion,
53 intermittent fixed portion,
54 pinch joining portion (joining portion),
541 front pinch joining portion, 542 back pinch joining portion

## Claims

1. An underpants-shaped absorbent article having a vertical direction, a lateral direction, and a front-back direction that intersect each other,
the underpants-shaped absorbent article comprising:
an absorbent main body (10) that is folded in the front-back direction at a vertically lower end portion,
the absorbent main body (10) including an absorbent core (11), a skin-side sheet (12) and a pair of leak-proof wall portions (40),
the absorbent core (11) being arranged extending in the vertical direction,
the skin-side sheet (12) being arranged on a wearer skin side with respect to the absorbent core (11);
a front waist portion (20) that is joined to an upper end portion of the absorbent main body (10) on a front side in the front-back direction; and
a back waist portion (30) that is joined to an upper end portion of the absorbent main body (10) on a back side in the front-back direction,
a lower end portion of the front waist portion (20) not including an inclined elastic member that is inclined downward and inward in the lateral direction,
the absorbent main body (10) including an elastic member (41) in two lateral side portions of the absorbent main body (10),
the elastic member (41) being capable of stretching and contracting in the vertical direction,
each of the pair of leak-proof wall portions (40) being capable of rising up on the skin side due to the elastic member (41),
the absorbent main body (10) including a front-end-portion fixed portion (51) in the upper end portion on the front side,
the front-end-portion fixed portion (51) fixing each of the leak-proof wall portions (40) in a manner that the leak-proof wall portion (40) is not capable of rising up,
the absorbent main body (10) including a back-end-portion fixed portion (52) in the upper end portion on the back side,
the back-end-portion fixed portion (52) fixing each of the leak-proof wall portions (40) in a manner that the leak-proof wall portion (40) is not capable of rising up,
the absorbent main body (10) including an intermittent fixed portion (53) at a position spaced apart from the front-end-portion fixed portion (51) and the back-end-portion fixed portion (52) in the vertical direction,
the intermittent fixed portion (53) fixing each of the leak-proof wall portions (40) to the skin-side sheet (12),
in a state where the underpants-shaped absorbent article is unfolded and stretched in the vertical direction, a vertical center (CL2) of the intermittent fixed portion (53) is on a front-waist-portion side with respect to a vertical center (CL1) of the underpants-shaped absorbent article.

2. The underpants-shaped absorbent article according to claim 1, wherein
the absorbent core (11) has a narrow portion (111) in a central portion in a longitudinal direction of the absorbent core (11),
a lateral width in the narrow portion (111) is smaller than in end portions of the absorbent core (11),
at least a part of the intermittent fixed portion (53) is arranged side-by-side with the narrow portion (111) in the lateral direction and outward in the lateral direction with respect to the narrow portion (111) .

3. The underpants-shaped absorbent article according to claim 1 or 2, wherein
in the state where the underpants-shaped absorbent article is unfolded and stretched in the vertical direction,
the intermittent fixed portion (53) is entirely on the front-waist-portion side with respect to the vertical center (CL1) of the underpants-shaped absorbent article.

4. The underpants-shaped absorbent article according to any one of claims 1 to 3, wherein
each of the leak-proof wall portions (40) has a base region (401) and a leading-end region (402),
the base region (401) being a region that extends from an inward end (53a) of the intermittent fixed portion (53) toward a base (43a) of the leak-proof wall portion (40) in the lateral direction,
the leading-end region (402) being a region that extends from the base region (401) toward a leading end (42a) of the leak-proof wall portion (40), and
the leading-end region (402) has a joining portion (54) at a location laterally inward with respect to the leading end (42a) of the leak-proof wall portion (40),
the joining portion (54) being a portion in which at least a portion of mutually opposing surfaces are joined to each other.

5. The underpants-shaped absorbent article according to claim 4, wherein
in the state where the underpants-shaped absorbent article is unfolded and stretched in the vertical direction,
the joining portion (54) is at a location not overlapped with the intermittent fixed portion (53) in the vertical direction.

6. The underpants-shaped absorbent article according to claim 4 or 5, wherein
concerning a portion of the absorbent main body (10) which is located between the front waist portion (20) and the back waist portion (30) and where at least either one of the intermittent fixed portion (53) and the joining portion (54) are arranged,
in the state where the underpants-shaped absorbent article is unfolded and stretched in the vertical direction, a vertical length of the portion of the absorbent main body (10) is longer than a vertical length of a remaining portion of the absorbent main body (10) which is located between the front waist portion (20) and the back waist portion (30).

7. The underpants-shaped absorbent article according to any one of claims 4 to 6, wherein
in the state where the underpants-shaped absorbent article is unfolded and stretched in the vertical direction,
a vertical length (L1) of the intermittent fixed portion (53) is shorter than a vertical length (L2, L3) of the joining portion (54) .

8. The underpants-shaped absorbent article according to any one of claims 1 to 7, wherein
the intermittent fixed portion (53) is arranged inward with respect to lateral side ends (10a) of the absorbent main body (10).

## Patentansprüche

1. Unterhosen-förmiger absorbierender Artikel, der eine vertikale Richtung, eine laterale Richtung und eine Vorn-Hinten-Richtung aufweist, die einander schneiden,
wobei der Unterhosen-förmige absorbierende Artikel Folgendes umfasst:
einen absorbierenden Hauptkörper (10), der in der Vorn-Hinten-Richtung an einem vertikal unteren Endteil gefaltet ist,
wobei der absorbierende Hauptkörper (10) einen Saugkern (11), eine Hautseitenlage (12) und ein Paar von auslaufsicheren Wandteilen (40) einschließt,
wobei der Saugkern (11) angeordnet ist, dass er sich in der vertikalen Richtung erstreckt,
die Hautseitenlage (12) auf einer Hautseite des Trägers in Bezug auf den Saugkern (11) angeordnet ist;
einen vorderen Taillenteil (20), der mit einem oberen Endteil des absorbierenden Hauptkörpers (10) auf einer vorderen Seite in der Vorn-Hinten-Richtung verbunden ist; und
einen hinteren Taillenteil (30), der mit einem oberen Endteil des absorbierenden Hauptkörpers (10) auf einer hinteren Seite in der Vorn-Hinten-Richtung verbunden ist,
einen unteren Endteil des vorderen Taillenteils (20), der kein abgeschrägtes elastisches Element einschließt, das nach unten und nach innen in der lateralen Richtung abgeschrägt ist,
wobei der absorbierende Hauptkörper (10) ein elastisches Element (41) in zwei lateralen Seitenteilen des absorbierenden Hauptkörpers (10) einschließt,
wobei sich das elastische Element (41) in der vertikalen Richtung dehnen und zusammenziehen kann,
wobei sich jeder des Paars von auslaufsicheren Wandteilen (40) auf der Hautseite aufgrund des elastischen Elements (41) aufstellen kann,
der absorbierende Hauptkörper (10) einen fixierten Teil des vorderen Endteils (51) in dem oberen Endteil auf der vorderen Seite einschließt,
wobei der fixierte Teil des vorderen Endteils (51) jeden der auslaufsicheren Wandteile (40) in einer Weise fixiert, dass sich der auslaufsichere Wandteil (40) nicht aufstellen kann,
wobei der absorbierende Hauptkörper (10) einen fixierten Teil des hinteren Endteils (52) in dem oberen Endteil auf der hinteren Seite einschließt,
wobei der fixierte Teil des hinteren Endteils (52) jeden der auslaufsicheren Wandteile (40) in einer Weise fixiert, dass sich der auslaufsichere Wandteil (40) nicht aufstellen kann,
wobei der absorbierende Hauptkörper (10) einen intermittierenden fixierten Teil (53) an einer Position einschließt, die von dem fixierten Teil des vorderen Endteils (51) und dem fixierten Teil des hinteren Endteils (52) in der vertikalen Richtung beabstandet ist,
wobei der intermittierende fixierte Teil (53) jeden der auslaufsicheren Wandteile (40) an der Hautseitenlage (12) fixiert,
wobei in einem Zustand, wo der Unterhosen-förmige absorbierende Artikel in der vertikalen Richtung entfaltet und gedehnt ist, eine vertikale Mitte (CL2) des intermittierenden fixierten Teils (53) auf einer vorderen Taillenteilseite in Bezug auf eine vertikale Mitte (CL1) des Unterhosen-förmigen absorbierenden Artikels vorliegt.

2. Unterhosen-förmiger absorbierender Artikel nach Anspruch 1, wobei
der Saugkörper (11) einen schmalen Teil (111) in einem mittleren Teil in einer Längsrichtung des Saugkerns (11) aufweist,
eine laterale Breite in dem schmalen Teil (111) kleiner ist als in Endteilen des Saugkerns (11),
zumindest ein Teil des intermittierenden fixierten Teils (53) neben dem schmalen Teil (111) in der lateralen Richtung und außenliegend in der lateralen Richtung in Bezug auf den schmalen Teil (111) angeordnet ist.

3. Unterhosen-förmiger absorbierender Artikel nach Anspruch 1 oder 2, wobei
in dem Zustand, wo der Unterhosen-förmige absorbierende Artikel in der vertikalen Richtung entfaltet und gedehnt ist,
der intermittierende fixierte Teil (53) vollständig auf der vorderen Taillenteilseite in Bezug auf die vertikale Mitte (CL1) des Unterhosen-förmigen absorbierenden Artikels vorliegt.

4. Unterhosen-förmiger absorbierender Artikel nach einem der Ansprüche 1 bis 3, wobei
jeder der auslaufsicheren Wandteile (40) einen Basisbereich (401) und einen Bereich des führenden Endes (402) aufweist,
wobei der Basisbereich (401) ein Bereich ist, der sich von einem innenliegenden Ende (53a) des intermittierenden fixierten Teils (53) in Richtung einer Basis (43a) des auslaufsicheren Wandteils (40) in der lateralen Richtung erstreckt,
wobei der Bereich des führenden Endes (402) ein Bereich ist, der sich von dem Basisbereich (401) in Richtung eines führenden Endes (42a) des auslaufsicheren Wandteils (40) erstreckt, und
der Bereich des führenden Endes (402) einen Verbindungsteil (54) an einer Stelle lateral innenliegend in Bezug auf das führende Ende (42a) des auslaufsicheren Wandteils (40) aufweist,
wobei der Verbindungsteil (54) ein Teil ist, in dem zumindest ein Teil von gegenseitig gegenüberliegenden Oberflächen miteinander verbunden sind.

5. Unterhosen-förmiger absorbierender Artikel nach Anspruch 4, wobei
in dem Zustand, wo der Unterhosen-förmige absorbierende Artikel in der vertikalen Richtung entfaltet und gedehnt ist,
der Verbindungsteil (54) an einer Stelle vorliegt, die nicht mit dem intermittierenden fixierten Teil (53) in der vertikalen Richtung überlappt ist.

6. Unterhosen-förmiger absorbierender Artikel nach Anspruch 4 oder 5, wobei
bezüglich eines Teils des absorbierenden Hauptkörpers (10), der sich zwischen dem vorderen Taillenteil (20) und dem hinteren Taillenteil (30) befindet und wo zumindest einer des intermittierenden fixierten Teils (53) und des Verbindungsteils (54) angeordnet ist,
in dem Zustand, wo der Unterhosen-förmige absorbierende Artikel in der vertikalen Richtung entfaltet und gedehnt ist, eine vertikale Länge des Teils des absorbierenden Hauptkörpers (10) länger ist als eine vertikale Länge eines verbleibenden Teils des absorbierenden Hauptkörpers (10), der sich zwischen dem vorderen Taillenteil (20) und dem hinteren Taillenteil (30) befindet.

7. Unterhosen-förmiger absorbierender Artikel nach einem der Ansprüche 4 bis 6, wobei
in dem Zustand, wo der Unterhosen-förmige absorbierende Artikel in der vertikalen Richtung entfaltet und gedehnt ist,
eine vertikale Länge (L1) des intermittierenden fixierten Teils (53) kürzer ist als eine vertikale Länge (L2, L3) des Verbindungsteils (54).

8. Unterhosen-förmiger absorbierender Artikel nach einem der Ansprüche 1 bis 7, wobei
der intermittierende fixierte Teil (53) innenliegend in Bezug auf laterale Seitenenden (10a) des absorbierenden Hauptkörpers (10) angeordnet ist.

## Revendications

1. Article absorbant en forme de culotte ayant une direction allant dans le sens de la verticale, une direction allant dans le sens latéral, et une direction allant d'avant en arrière qui se croisent les unes les autres,
l'article absorbant en forme de culotte comportant :
un corps principal absorbant (10) qui est plié dans la direction allant d'avant en arrière au niveau d'une partie d'extrémité inférieure à la verticale,
le corps principal absorbant (10) comprenant une partie centrale absorbante (11), une feuille côté orienté vers la peau (12) et une paire de parties formant paroi antifuite (40) ,
la partie centrale absorbante (11) étant agencée pour s'étendre dans la direction allant dans le sens de la verticale,
la feuille côté orienté vers la peau (12) étant agencée sur le côté orienté vers la peau d'un utilisateur par rapport à la partie centrale absorbante (11) ;
une partie avant au niveau de la taille (20) qui est assemblée sur une partie d'extrémité supérieure du corps principal absorbant (10) sur un côté avant dans la direction allant d'avant en arrière ; et
une partie arrière au niveau de la taille (30) qui est assemblée sur une partie d'extrémité supérieure du corps principal absorbant (10) sur un côté arrière dans la direction allant d'avant en arrière,
une partie d'extrémité inférieure de la partie avant au niveau de la taille (20) ne comprenant pas un élément élastique incliné qui est incliné vers le bas et vers l'intérieur dans la direction allant dans le sens latéral,
le corps principal absorbant (10) comprenant un élément élastique (41) dans deux parties côté latéral du corps principal absorbant (10),
l'élément élastique (41) étant en mesure de s'étirer et de se contracter dans la direction allant dans le sens de la verticale,
chacune de la paire de parties formant paroi antifuite (40) étant en mesure de se surélever sur le côté orienté vers la peau en raison de l'élément élastique (41),
le corps principal absorbant (10) comprenant une partie fixe de partie d'extrémité avant (51) dans la partie d'extrémité supérieure sur le côté avant,
la partie fixe de partie d'extrémité avant (51) fixant chacune des parties formant paroi antifuite (40) d'une telle manière que la partie formant paroi antifuite (40) n'est pas en mesure de se surélever,
le corps principal absorbant (10) comprenant une partie fixe de partie d'extrémité arrière (52) dans la partie d'extrémité supérieure sur le côté arrière,
la partie fixe de partie d'extrémité arrière (52) fixant chacune des parties formant paroi antifuite (40) d'une telle manière que la partie formant paroi antifuite (40) n'est pas en mesure de se surélever,
le corps principal absorbant (10) comprenant une partie fixe intermittente (53) au niveau d'une position espacée par rapport à la partie fixe de partie d'extrémité avant (51) et par rapport à la partie fixe de partie d'extrémité arrière (52) dans la direction allant dans le sens de la verticale,
la partie fixe intermittente (53) fixant chacune des parties formant paroi antifuite (40) sur la feuille côté orienté vers la peau (12),
dans un état dans lequel l'article absorbant en forme de culotte est déplié et étendu dans la direction allant dans le sens de la verticale, un centre vertical (CL2) de la partie fixe intermittente (53) est sur un côté partie avant au niveau de la taille par rapport à un centre vertical (CL1) de l'article absorbant en forme de culotte.

2. Article absorbant en forme de culotte selon la revendication 1, dans lequel
la partie centrale absorbante (11) a une partie étroite (111) dans une partie centrale dans une direction allant dans le sens longitudinal de la partie centrale absorbante (11),
une largeur latérale dans la partie étroite (111) est inférieure à la largeur dans les parties d'extrémité de la partie centrale absorbante (11),
au moins une partie de la partie fixe intermittente (53) est agencée côte-à-côte par rapport à la partie étroite (111) dans la direction allant dans le sens latéral et vers l'extérieur dans la direction allant dans le sens latéral par rapport à la partie étroite (111).

3. Article absorbant en forme de culotte selon la revendication 1 ou la revendication 2, dans lequel
dans l'état dans lequel l'article absorbant en forme de culotte est déplié et étiré dans la direction allant dans le sens de la verticale,
la partie fixe intermittente (53) est entièrement sur le côté partie avant au niveau de la taille par rapport au centre vertical (CL1) de l'article absorbant en forme de culotte.

4. Article absorbant en forme de culotte selon l'une quelconque des revendications 1 à 3, dans lequel
chacune des parties formant paroi antifuite (40) a une région de base (401) et une région d'extrémité avant (402),
la région de base (401) étant une région qui s'étend depuis une extrémité allant vers l'intérieur (53a) de la partie fixe intermittente (53) vers une base (43a) de la partie formant paroi antifuite (40) dans la direction allant dans le sens latéral,
la région d'extrémité avant (402) étant une région qui s'étend depuis la région de base (401) vers une extrémité avant (42a) de la partie formant paroi antifuite (40), et
la région d'extrémité avant (402) a une partie d'assemblage (54) au niveau d'un emplacement se trouvant vers l'intérieur dans le sens latéral par rapport à l'extrémité avant (42a) de la partie formant paroi antifuite (40),
la partie d'assemblage (54) étant une partie dans laquelle au moins une partie de surfaces mutuellement opposées sont assemblées l'une par rapport à l'autre.

5. Article absorbant en forme de culotte selon la revendication 4, dans lequel
dans l'état dans lequel l'article absorbant en forme de culotte est déplié et étiré dans la direction allant dans le sens de la verticale,
la partie d'assemblage (54) se trouve au niveau d'un emplacement qui n'est pas chevauché par la partie fixe intermittente (53) dans la direction allant dans le sens de la verticale.

6. Article absorbant en forme de culotte selon la revendication 4 ou la revendication 5, dans lequel
concernant une partie du corps principal absorbant (10) qui se trouve entre la partie avant au niveau de la taille (20) et la partie arrière au niveau de la taille (30) et où au moins l'une ou l'autre parmi la partie fixe intermittente (53) et la partie d'assemblage (54) sont agencées,
dans l'état dans lequel l'article absorbant en forme de culotte est déplié et étiré dans la direction allant dans le sens de la verticale, une longueur verticale de la partie du corps principal absorbant (10) est plus longue qu'une longueur verticale d'une partie restante du corps principal absorbant (10) qui se trouve entre la partie avant au niveau de la taille (20) et la partie arrière au niveau de la taille (30).

7. Article absorbant en forme de culotte selon l'une quelconque des revendications 4 à 6, dans lequel
dans l'état dans lequel l'article absorbant en forme de culotte est déplié et étiré dans la direction allant dans le sens de la verticale,
une longueur verticale (L1) de la partie fixe intermittente (53) est plus courte qu'une longueur verticale (L2, L3) de la partie d'assemblage (54).

8. Article absorbant en forme de culotte selon l'une quelconque des revendications 1 à 7, dans lequel
la partie fixe intermittente (53) est agencée vers l'intérieur par rapport à des extrémités côté latéral (10a) du corps principal absorbant (10).
